# EUROPÄISCHE PATENTSCHRIFT

(11) **EP 2 200 970 B1**
(45) Veröffentlichungstag und Bekanntmachung des Hinweises auf die Patenterteilung: **06.01.2016**
(21) Anmeldenummer: 08803396.4
(22) Anmeldetag: 29.08.2008
(51) Int. Cl.: C07C 223/02, C07D 295/10, C07D 295/108, C08G 18/28, C08G 18/32, C08G 18/36, C08G 18/50, C08G 18/66

(54) **HYDROXYLGRUPPEN AUFWEISENDE ALDEHYDE**
ALDEHYDES CONTAINING HYDROXYL GROUPS
ALDÉHYDES PRÉSENTANT DES GROUPES HYDROXYLE

(30) Priorität: 31.08.2007 EP 07115439
(43) Veröffentlichungstag der Anmeldung: 30.06.2010
(73) Patentinhaber: Sika Technology AG, 6340 Baar (CH)
(72) Erfinder: BURCKHARDT, Urs, CH-8049 Zürich (CH)
(74) Vertreter: Sika Patent Attorneys
(86) Internationale Anmeldenummer: PCT/EP2008/061398
(87) Internationale Veröffentlichungsnummer: WO 2009/027510

(56) Entgegenhaltungen:
- EP-A- 0 046 288
- EP-A- 0 254 177
- EP-A- 1 524 282
- EP-A- 1 717 253
- WO-A-2006/074051
- GB-A- 1 193 347
- US-A- 1 824 676
- US-A- 3 698 898
- US-A- 4 224 417
- JOHNSON, P.Y. ET AL.: "The chemistry of hindered systems. 2. The acyloin reaction - an approach to regiospecifically hydroxylated tetramethylazacycloheptane systems" J. ORG. CHEM., Bd. 41, Nr. 10, 1976, Seiten 1768-1773, XP002468360
- MANNICH, C. ET AL.: "A synthesis of N-substituted .alpha.-amino aldehydes Ueber eine Synthese von N-substituierten Beta-Amino-aldehyden" BERICHTE DER DEUTSCHEN CHEMISCHEN GESELLSCHAFT ABTEILUNG B: ABHANDLUNGEN, Bd. 65B, 1932, Seiten 378-385, XP009095805 ISSN: 0365-9488

## Beschreibung

Die Erfindung betrifft das Gebiet der Aldehyde und das Gebiet der Polyurethane.

### Stand der Technik

Die α-Aminoalkylierung von Aldehyden mit Formaldehyd und primären oder sekundären aliphatischen Aminen wird von C. Mannich, beispielsweise in US 1,824,676, offenbart. Die auf diese Weise erhältlichen β-Amino-Aldehyde werden auch als "Mannich-Basen" bezeichnet. Als vielseitige Synthesebausteine haben sie eine breite Verwendung gefunden.

Für gewisse Verwendungen wäre es vorteilhaft, Aldehyde mit weiteren funktionellen Gruppen zur Verfügung zu haben. Insbesondere in der Polyurethanchemie besteht ein Bedarf an Aldehyden, welche gegenüber Isocyanatgruppen reaktionsfähige funktionelle Gruppen aufweisen und damit kovalent in ein Polyurethanpolymer eingebaut werden können.

Der Einsatz von Polyaldiminen in Polyurethanzusammensetzungen ist beispielsweise bekannt aus EP0254177 oder aus EP1524282. Die Herstellung von Mannichbasen ist beispielweise beschrieben in EP1717253.

### Darstellung der Erfindung

Aufgabe der vorliegenden Erfindung ist es daher, neue Aldehyde zur Verfügung zu stellen, die reaktive funktionelle Gruppen aufweisen, welche für weiterführende Reaktionen verfügbar sind.

Überraschenderweise wurde gefunden, dass Aldehyde der Formel (I) oder (V) diese Aufgabe lösen. Es handelt sich dabei um bisher nicht beschriebene, Hydroxylgruppen aufweisende Mannich-Basen. Es sind bei Raumtemperatur meist flüssige Verbindungen, welche kaum Geruch aufweisen und aus gut erhältlichen Grundstoffen in einem einfachen Verfahren herstellbar sind. Sie weisen tertiäre Aminogruppen von relativ niedriger Basizität auf und können in chemischen Reaktionssystemen katalytisch wirken. Ihre Hydroxylgruppen stehen für weiterführende Reaktionen, beispielsweise mit Isocyanatgruppen, zur Verfügung.

Diese Aldehyde sind beispielsweise geeignet zur Herstellung von Aldiminen, welche als Härter für härtbare Zusammensetzungen mit gegenüber Aminen reaktiven Gruppen, wie Epoxidgruppen, Anhydridgruppen oder insbesondere Isocyanatgruppen, eingesetzt werden können. In Isocyanatgruppen aufweisenden Zusammensetzungen werden die bei der Aushärtung aus den Aldiminen wieder freigesetzten Aldehyde über deren Hydroxylgruppen kovalent in das entstehende Polyurethanpolymer eingebaut und verbleiben so vollständig in der Zusammensetzung.

Ein weiterer Gegenstand der Erfindung sind Aldehyde der Formel (VII), welche Umsetzungsprodukte der beschriebenen Aldehyde darstellen.

Ein weiterer Gegenstand der Erfindung sind härtbare Zusammensetzungen enthaltend die beschriebenen Aldehyde.

Schliesslich bilden ein Verfahren zur Herstellung der Aldehyde, eine ausgehärtete Zusammensetzung, Verwendungen sowie ein mittels der Aldehyde verklebter Artikel weitere Gegenstände der vorliegenden Erfindung.

### Wege zur Ausführung der Erfindung

Gegenstand der Erfindung sind Aldehyde **ALD** der Formel (I), 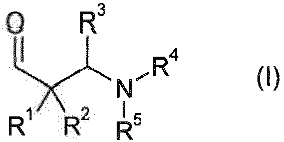 wobei
R¹ und R² entweder
unabhängig voneinander jeweils für einen einwertigen Kohlenwasserstoffrest mit 1 bis 12 C-Atomen stehen,
oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder eine Alkylgruppe oder eine Arylalkylgruppe oder eine Alkoxycarbonylgruppe, insbesondere mit 1 bis 12 C-Atomen, steht; und
R⁴ und R⁵ entweder
unabhängig voneinander jeweils für eine Methylgruppe oder einen einwertigen aliphatischen, cycloaliphatischen oder arylaliphatischen Rest mit 2 bis 20 C-Atomen stehen, welcher gegebenenfalls Hydroxylgruppen oder Aldehydgruppen aufweist, und welcher gegebenenfalls Heteroatome in der Form von Ether-Sauerstoff oder tertiärem Amin-Stickstoff enthält, mit der Massgabe, dass R⁴ mindestens eine Hydroxylgruppe aufweist und dass R⁴ und R⁵ zusammen mindestens zwei Hydroxylgruppen aufweisen,
oder
zusammen für einen mindestens zwei Hydroxylgruppen aufweisenden zweiwertigen aliphatischen Rest mit 4 bis 20 C-Atomen, der Teil eines, gegebenenfalls substituierten, heterocyclischen Rings mit 5 bis 8, bevorzugt 6, Ringatomen ist, wobei dieser Ring gegebenenfalls weitere Heteroatome in der Form von Ether-Sauerstoff oder tertiärem Amin-Stickstoff enthält und gegebenenfalls Aldehydgruppen aufweist, stehen.

Bevorzugt stehen R¹ und R² jeweils für eine Methylgruppe.

Bevorzugt steht R³ für ein Wasserstoffatom.

Bevorzugt stehen R⁴ und R⁵ jeweils für eine 2-Hydroxyethylgruppe oder für eine 2-Hydroxypropylgruppe.

Die am meisten bevorzugten Aldehyde **ALD** der Formel (I) enthalten in einer Ausführungsform einen Rest R⁴ mit zwei Hydroxylgruppen und einen Rest R⁵ ohne Hydroxylgruppe; oder in einer weiteren Ausführungsform einen Rest R^{4'} mit einer Hydroxylgruppe und einen Rest R⁵ mit einer Hydroxylgruppe.

Aldehyde **ALD** der Formel (I) sind insbesondere erhältlich als Produkt einer Mannich-Reaktion oder einer der Mannich-Reaktion analogen α-Aminoalkylierung, wie sie aus der Fachliteratur bekannt ist; sie können deshalb auch als Mannich-Basen bezeichnet werden.

In einer Ausführungsform werden ein Aldehyd **Y1** der Formel (II), ein Aldehyd **Y2** der Formel (III) und entweder ein sekundäres aliphatisches, mindestens zwei Hydroxylgruppen aufweisendes Amin **C** der Formel (IV) unter Wasserabspaltung zu einem Aldehyd **ALD** der Formel (I) oder ein sekundäres aliphatisches, mindestens eine Hydroxylgruppe aufweisendes Amin **C** der Formel (IV) unter Wasserabspaltung zu einem Aldehyd der Formel (V) umgesetzt.

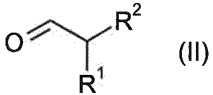

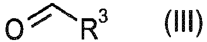

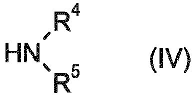

Diese Umsetzung kann entweder mit den freien Reagenzien **Y1, Y2** und **C** gemäss den Formeln (II), (III) und (IV) geführt werden, oder die Reagenzien können teilweise oder vollständig in derivatisierter Form eingesetzt werden. So kann der Aldehyd **Y1** beispielsweise als Enolat, als Enolether, insbesondere als Silylenolether, oder als Enamin eingesetzt werden. Der Aldehyd **Y2** kann beispielsweise in Form eines Oligomeren - im Fall von Formaldehyd insbesondere als 1,3,5-Trioxan oder als Paraformaldehyd - oder als Hydrat, Hemiacetal, Acetal, N,O-Acetal, Aminal oder Hemiaminal eingesetzt werden. Das sekundäre aliphatische Amin **C** schliesslich kann beispielsweise als Salz, insbesondere als Amin-Hydrochlorid oder als Amin-Hydrosulfat, oder als Silylamin eingesetzt werden. Es ist möglich, einen Teil der Reagenzien in freier Form und einen Teil in derivatisierter Form einzusetzen, oder nur von derivatisierten Formen auszugehen. Bei der Verwendung von Reagenzien in derivatisierter Form fällt der Aldehyd **ALD** unter Umständen ebenfalls in derivatisierter Form, beispielsweise als Salz, an; in diesem Fall kann er durch geeignete Aufarbeitung in die freie Form gemäss Formel (I) bzw. (V) übergeführt werden. Je nachdem kann es sinnvoll sein, in solchen Umsetzungsreaktionen zusätzlich Hilfsstoffe wie Lewissäuren oder Katalysatoren einzusetzen.

Weiterhin kann die Umsetzung als Eintopfreaktion geführt werden, in der alle drei Reagenzien gleichzeitig miteinander reagieren können; oder aber es kann ein stufenweises Vorgehen gewählt werden, indem zwei der Reagenzien vorgängig miteinander umgesetzt werden und das so erhaltene Zwischenprodukt anschliessend mit dem dritten Reagens umgesetzt wird, wobei das Zwischenprodukt isoliert werden kann oder nicht. Als solche Zwischenprodukte geeignet sind insbesondere Iminiumsalze, welche aus der Umsetzung eines Aldehyds **Y2,** in freier oder derivatisierter Form, mit einem Salz eines sekundären aliphatischen Amins **C** erhalten werden und welche sich mit einem Aldehyd **Y1,** in freier oder derivatisierter Form, zum entsprechenden Salz eines Aldehyds **ALD** der Formel (I) umsetzen lassen. Ein solcherart stufenweises Vorgehen kann den Vorteil haben, mildere Reaktionsbedingungen zu ermöglichen und damit eine höhere Produktausbeute zu liefern.

Weiterhin kann die Umsetzung unter Verwendung von Lösemitteln, insbesondere polaren Lösemitteln wie Wasser oder Alkoholen, durchgeführt werden, oder die Umsetzung kann ohne Verwendung von Lösemitteln erfolgen.

In einer bevorzugten Ausführungsform wird die Umsetzung mit allen Reagenzien in freier Form als Eintopfreaktion geführt und der Aldehyd **ALD** nach erfolgter Umsetzung durch Destillation gereinigt. Bevorzugt werden dabei keine organischen Lösemittel eingesetzt.

Als Aldehyd **Y1** der Formel (II) geeignet sind beispielsweise die folgenden: Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Iso-butyraldehyd.

Als Aldehyd **Y2** der Formel (III) geeignet sind beispielsweise die folgenden: Formaldehyd, Acetaldehyd, Propionaldehyd, Butyraldehyd, Isobutyraldehyd, Phenylacetaldehyd, Benzaldehyd und substituierte Benzaldehyde sowie Glyoxylsäureester, insbesondere Glyoxylsäureethylester. Bevorzugt ist Formaldehyd.

Als Amin C der Formel (IV) geeignet sind in einer Ausführungsform mindestens zwei Hydroxylgruppen aufweisende sekundäre aliphatische Amine **C1.**

Als "primäres Amin" bzw. "sekundäres Amin" bzw. "tertiäres Amin" werden im vorliegenden Dokument Verbindungen bezeichnet, welche primäre, bzw. sekundäre, bzw. tertiäre, Aminogruppen tragen. Der Begriff "primäre Aminogruppe" bezeichnet eine Aminogruppe in der Form einer NH₂-Gruppe, die an einen organischen Rest gebunden ist. Der Begriff "sekundäre Aminogruppe" bezeichnet eine Aminogruppe, in der das Stickstoffatom an zwei organische Reste gebunden ist, welche auch gemeinsam Teil eines Rings sein können. Der Begriff "tertiäre Aminogruppe" bezeichnet eine Aminogruppe, in der das Stickstoffatom (=tertiärer Amin-Stickstoff) an drei organische Reste gebunden ist, wobei zwei dieser Reste auch gemeinsam Teil eines Rings sein können.

Als "aliphatisch" wird im vorliegenden Dokument ein Amin oder eine Aminogruppe bezeichnet, worin das Stickstoffatom ausschliesslich an aliphatische, cycloaliphatische oder arylaliphatische Reste gebunden ist.

Geeignete Amine **C1** mit zwei Hydroxylgruppen sind insbesondere ausgewählt aus der Gruppe bestehend aus Diethanolamin, Dipropanolamin, Diisopropanolamin, 3-(2-Hydroxyethylamino)-1-propanol und 3-(2-Hydroxypropylamino)-1-propanol, N-Methyl-2,3-dihydroxypropylamin, 3,4-Dihydroxypyrrolidin, 2,5-Bis-(hydroxymethyl)-pyrrolidin, 2,6-Bis-(hydroxymethyl)-piperidin, 3,4- oder 3,5-Dihydroxy-piperidin, 2-(2,3-Dihydroxypropyl)-pyrrolidin und 2-(2,3-Dihydroxypropyl)-piperidin sowie die Umsetzungsprodukte aus Ammoniak mit zwei Molekülen, welche je eine Epoxidgruppe, insbesondere eine Glycidylether-Gruppe, aufweisen.

Geeignete Amine **C1** mit mehr als zwei Hydroxylgruppen sind beispielsweise die Folgenden: 2-(2,3-Dihydroxypropylamino)-ethanol, 3,4,5-Trihydroxy-piperidin, N,N-Bis-(2,3-dihydroxypropyl)-amin, 2,5-Bis-(2,3-dihydroxypropyl)-pyrrolidin und 2,6-Bis-(2,3-dihydroxypropyl)-piperidin.

Als Amin **C** der Formel (IV) geeignet sind weiterhin Amine **C3** mit zwei sekundären aliphatischen Aminogruppen und mindestens einer Hydroxylgruppe, wie beispielsweise 2-Hydroxymethyl-piperazin, 2-(2,3-Dihydroxypropyl)-piperazin, 2,3- oder 2,6-Bis-(hydroxymethyl)-piperazin und N,N'-Dimethylpropylen-2-ol-diamin. Die Amine **C3** werden mit jeweils der doppelten Menge Aldehyd **Y1** und Aldehyd **Y2** umgesetzt. Dabei entstehen Aldehyde **ALD** in der Form von Hydroxylgruppen aufweisenden Aldehyden der Formel (V), 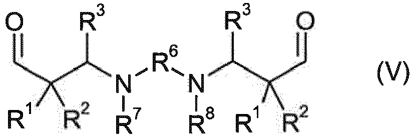 wobei
R⁶ für einen zweiwertigen aliphatischen, cycloaliphatischen oder arylaliphatischen Rest mit 2 bis 20 C-Atomen, welcher gegebenenfalls Hydroxylgruppen aufweist, und welcher gegebenenfalls Heteroatome in der Form von Ether-Sauerstoff oder tertiärem Amin-Stickstoff enthält, steht;
R⁷ und R⁸ entweder
unabhängig voneinander jeweils für eine Methylgruppe oder einen einwertigen aliphatischen, cycloaliphatischen oder arylaliphatischen Rest mit 2 bis 20 C-Atomen, welcher gegebenenfalls Hydroxylgruppen aufweist, und welcher gegebenenfalls Heteroatome in der Form von Ether-Sauerstoff oder tertiärem Amin-Stickstoff enthält, stehen,
oder
zusammen für einen mindestens eine Hydroxylgruppe aufweisenden zweiwertigen aliphatischen Rest mit 4 bis 20 C-Atomen stehen, welcher zusammen mit N-R⁶-N einen heterocyclischen Ring mit 5 bis 8, bevorzugt 6, Ring-Atomen bildet, wobei dieser Ring gegebenenfalls weitere Heteroatome in der Form von Ether-Sauerstoff oder tertiärem Amin-Stickstoff enthält,
mit der Massgabe, dass mindestens einer der Reste R⁶, R⁷ und R⁸ mindestens eine Hydroxylgruppe aufweist,
und R¹, R² und R³ die bereits genannten Bedeutungen aufweisen.

In einer weiteren Ausführungsform können Aldehyde **ALD** der Formel (I) auch über primäre aliphatische, mindestens eine Hydroxylgruppe aufweisende Amine **C'** der Formel (IV') hergestellt werden, indem diese mit einem Aldehyd **Y1** und einem Aldehyd **Y2** im Molverhältnis 1:2:2 unter Wasserabspaltung umgesetzt werden. Dabei entstehen Aldehyde **ALD** in der Form von Aldehyden der Formel (VI).

H₂N-R⁴ (IV')

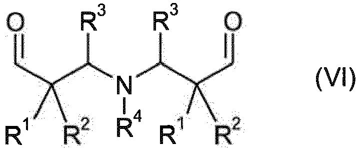

Als Amin C' der Formel (IV') geeignet sind primäre aliphatische Amine mit einer oder mehreren Hydroxylgruppen, insbesondere solche, welche ausgewählt sind aus der Gruppe bestehend aus Aminoalkoxylaten wie 2-Aminoethanol, 3-Amino-1-propanol, 1-Amino-2-propanol, 2-Amino-1-butanol, 3-(2-Hydroxyethoxy)-propylamin, 2-(2-Aminoethoxy)ethanol, sowie 4-(2-Aminoethyl)-2-hydroxyethylbenzol und 3-Aminomethyl-3,5,5-trimethyl-cyclohexanol.

Bevorzugte Aldehyde **ALD** sind 3-(N-Bis(2-hydroxyethyl)amino)-2,2-dimethyl-propanal und 3-(N-Bis(2-hydroxy-2-methylethyl)amino)-2,2-dimethylpropanal.

Die Aldehyde **ALD** der Formel (I) bzw. die Aldehyde der Formel (V) lassen sich auf die beschriebene Weise aus gut erhältlichen Grundstoffen in einem einfachen Verfahren herstellen. Sie weisen eine Reihe von besonderen Eigenschaften auf. So besitzen sie eine gute thermische Stabilität, weil das C-Atom in α-Stellung zur Aldehydgruppe kein Wasserstoffatom trägt und deshalb die Eliminierung eines sekundären Amins unter Bildung eines Alkens nicht möglich ist. Ferner weisen sie eine überraschend gute Stabilität gegenüber Oxidation durch Luftsauerstoff auf. Weiterhin ist ihre Basizität überraschenderweise deutlich niedriger als für aliphatische Amine ähnlicher Struktur erwartet; der für die konjugierte Säure eines entsprechenden Aldehyds gemessene *p*Kₐ-Wert liegt um ungefähr 2 Einheiten tiefer als jener der konjugierten Säure des zur Herstellung dieses Aldehyds eingesetzten primären oder sekundären Amins **C.** Diese überraschenden Eigenschaften stehen eventuell in Zusammenhang mit einer intramolekularen 1,4-Wechselwirkung zwischen Amin- und Aldehydgruppe (Orbitalüberlappung zwischen dem freien Elektronenpaar des Stickstoffs und dem π- bzw. π*-Orbital des Carbonyls), wie sie von P.Y. Johnson et al. (J. Org. Chem., Vol. 40, Nr. 19, 1975; Seiten 2710-2720) anhand von NMR- und UVspektroskopischen Untersuchungen an β-Aminoaldehyden postuliert wurde.

Schliesslich weisen diese Aldehyde, auch bei relativ niedrigem Molekulargewicht, keinen oder nur einen geringen Geruch auf. Diese für Aldehyde überraschende Eigenschaft der geringen Geruchsintensität kommt einerseits daher, dass die Aldehyde aufgrund der vorhandenen OH-Gruppen relativ wenig flüchtig sind. Weiterhin wird der geringe Geruch vermutlich begünstigt durch die erwähnte intramolekulare 1,4-Wechselwirkung und durch die sterische Hinderung der Aldehydgruppe, welche an einem tertiären C-Atom steht.

Die Aldehyde **ALD** der Formel (I) bzw. die Aldehyde der Formel (V) lassen sich sehr breit verwenden.

Sie können als Katalysatoren für chemische Reaktionssysteme eingesetzt werden, beispielsweise in härtbaren, Isocyanatgruppen aufweisenden Zusammensetzungen, insbesondere um deren Aushärtungszeit zu verkürzen.

Ihre Aldehydgruppen und ihre Hydroxylgruppen ermöglichen Folgereaktionen zum Aufbau von weiter funktionalisierten Reaktionsprodukten der Aldehyde. Sie können beispielsweise mit gegenüber Hydroxylgruppen reaktiven Verbindungen zu Aldehydgruppen aufweisenden Reaktionsprodukten umgesetzt werden. Geeignete gegenüber Hydroxylgruppen reaktive Verbindungen sind beispielsweise Isocyanate, Epoxide, Anhydride und Carbonsäuren. So lassen sich beispielsweise Isocyanatgruppen aufweisende Verbindungen durch die Umsetzung mit Hydroxylgruppen der Aldehyde zu Aldehydgruppen aufweisenden Verbindungen, beispielsweise Aldehydgruppen aufweisenden Polyurethanpolymeren oder Aldehydgruppen aufweisenden Polyisocyanaten, umsetzen.

Weiterhin können die Aldehydgruppen der erfindungsgemässen Aldehyde mit Verbindungen, welche primäre Aminogruppen aufweisen, zu Aldiminen umgesetzt werden. Aldimine können als geschützte Aldehydgruppen und / oder geschützte ("blockierte") Aminogruppen verwendet werden. Einerseits sind auf diese Weise hydroxyfunktionelle Monoaldimine erhältlich, welche ihrerseits mit gegenüber Hydroxylgruppen reaktiven Verbindungen zu weiterführenden Reaktionsprodukten umgesetzt werden können. Andererseits sind auf diese Weise hydroxyfunktionelle Polyaldimine erhältlich, welche beispielsweise in der Polyurethanchemie als Härter verwendet werden können. Werden die Polyaldimine in Anwesenheit von Feuchtigkeit mit Polyisocyanaten in Kontakt gebracht, so reagieren die durch Hydrolyse der Aldiminogruppen formal frei werdenden primären Aminogruppen mit den Isocyanatgruppen unter Freisetzung der Aldehyde, während deren Hydroxylgruppen ebenfalls mit Isocyanatgruppen reagieren. Die freigesetzten Aldehyde werden schliesslich kovalent in das bei der Aushärtung entstehende Polymer eingebaut, was sehr vorteilhaft ist. Durch den Einbau der Aldehyde verursachen diese in der Zusammensetzung keine nachteiligen Effekte, wie beispielsweise erhöhten Schwund, Immissionen in die Umgebungsluft, insbesondere von unangenehmen Gerüchen, oder Migrationeffekte wie Ausschwitzungen; ebenso haben sie keinen nachteiligen Einfluss auf die mechanischen Eigenschaften der Zusammensetzung, beispielsweise indem sie weichmachend wirken oder die Beständigkeit der Zusammensetzung gegenüber Umwelteinflüssen wie Hitze oder UV-Strahlung vermindern würden. Wie bereits erwähnt, sind die Aldehyde ALD bevorzugt, da sie mindestens zwei Hydroxylgruppen tragen und somit bei der Aushärtung von Isocyanatgruppen aufweisenden Zusammensetzungen als mindestens difunktionelle Härter unter Kettenverlängerung oder Vernetzung kovalent in das entstehende Polymer eingebaut werden. Die erwähnte relativ geringe Basizität der Aldehyde ist für eine solche Verwendung vorteilhaft, da stark basische tertiäre Amine die direkte Reaktion der Isocyanatgruppen, insbesondere mit Wasser, übermässig beschleunigen können, was sich bei der Aushärtung von Polyurethanen störend auswirken kann.

Weiterhin können die erfindungsgemässen Aldehyde als Startermoleküle für Polyalkoxylierungen eingesetzt werden. Auf diese Weise sind, je nach Anzahl vorhandener Hydroxylgruppen des verwendeten Aldehyds, Aldehydgruppen aufweisende Monole oder Polyole erhältlich. So sind mindestens eine Aldehydgruppe aufweisende Polyole aus der Polyalkoxylierung eines Aldehyds **ALD** der Formel (I) erhältlich.

Schliesslich können die Aldehyde als Quelle für kationische Verbindungen verwendet werden, indem die tertiären Aminogruppen teilweise oder ganz zu Ammoniumgruppen protoniert oder zu quaternären Ammoniumgruppen alkyliert werden. Durch Protonierung oder Alkylierung von Aldehyden **ALD** der Formel (I) sind Aldehyde der Formel (VII) zugänglich, 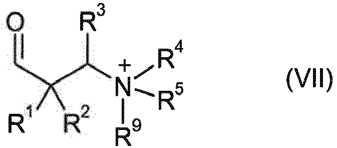 wobei
R⁹ für ein Wasserstoffatom oder für einen Alkyl-, Cycloalkyl- oder Arylalkylrest mit 1 bis 20 C-Atomen steht;
und R¹, R², R³, R⁴ und R⁵ die bereits genannten Bedeutungen aufweisen.

Zur Protonierung der erfindungsgemässen Aldehyde können beliebige Brønsted-Säuren eingesetzt werden, beispielsweise Salzsäure, Schwefelsäure, Phosphorsäure, Carbonsäuren wie Essigsäure oder Benzoesäure sowie Sulfonsäuren wie Methansulfonsäure oder p-Toluolsulfonsäure. Zur Alkylierung der Aldehyde können bekannte Alkylierungsmittel, insbesondere Methylierungsmittel, eingesetzt werden, beispielsweise Methyliodid, Dimethylsulfat, Dimethylphosphonat, Diazomethan, Fluorschwefelsäuremethylester oder Trimethyloxonium-tetrafluoroborat.

Es ist dem Fachmann klar, dass zu einem kationischen Aldehyd der Formel (VII) auch ein Anion gehört, das die positive Ladung des Aldehyds ausgleicht.

Die hier erwähnten Reaktionsprodukte ausgehend von den beschriebenen Aldehyden sind nur eine kleine Auswahl der Möglichkeiten von vorteilhaften Umsetzungsprodukten derselben und sollen die Anwendung der Aldehyde in keiner Weise einschränken.

Ein weiterer Gegenstand der Erfindung sind härtbare Zusammensetzungen, enthaltend mindestens ein Polyisocyanat und mindestens ein Aldehyd **ALD** der Formel (I), mindestens ein Aldehyd der Formel (V) oder mindestens ein Aldehyd der Formel (VII).

Der Begriff "Polyisocyanat" umfasst im vorliegenden Dokument Verbindungen mit zwei oder mehr Isocyanatgruppen, unabhängig davon, ob es sich dabei um monomere Diisocyanate, oligomere Polyisocyanate oder Isocyanatgruppen aufweisende Polymere mit einem relativ hohen Molekulargewicht handelt.

Als Aldehyd **ALD** der Formel (I) geeignet sind insbesondere die vorgängig detailliert beschriebenen Aldehyde **ALD** der Formel (I), respektive die bevorzugten Ausführungsformen davon. Geeignete Aldehyde der Formel (VII) wurden vorgängig bereits beschrieben.

Bevorzugt sind härtbare Zusammensetzungen enthaltend mindestens ein Polyisocyanat und mindestens ein Aldehyd **ALD** der Formel (I).

In einer Ausführungsform ist die härtbare Zusammensetzung einkomponentig.

Als "einkomponentig" wird im vorliegenden Dokument eine härtbare Zusammensetzung bezeichnet, bei welcher alle Bestandteile der Zusammensetzung vermischt im gleichen Gebinde gelagert werden, und welche bei Raumtemperatur über einen längeren Zeitraum lagerstabil ist, sich also in ihren Anwendungs- oder Gebrauchseigenschaften durch die Lagerung nicht oder nur unwesentlich verändert, und welche nach der Applikation durch die Einwirkung von Feuchtigkeit und / oder Wärme aushärtet.

Die einkomponentige härtbare Zusammensetzung umfasst mindestens ein Polyisocyanat, dessen Isocyanatgruppen insbesondere als blockierte Isocyanatgruppen vorliegen.

Unter einer "blockierten Isocyanatgruppe" wird im vorliegenden Dokument eine Isocyanatgruppe verstanden, welche durch die vorgängige Umsetzung einer freien Isocyanatgruppe mit einem aus dem Stand der Technik bekannten Blockierungsmittel, beispielsweise einem Phenol, einem Ketoxim, einem Pyrazol, einem Lactam oder einem Malonsäurediester, in ihrer Reaktivität gegenüber Nucleophilen so stark reduziert ist, dass sie zusammen mit geeigneten Härtern bei Raumtemperatur lagerstabil ist und erst unter der Einwirkung von Hitze und / oder Feuchtigkeit mit diesen Härtern zu reagieren beginnt, wobei das Blockierungsmittel je nach Typ freigesetzt oder nicht freigesetzt wird.

Geeignete Polyisocyanate mit blockierten Isocyanatgruppen sind kommerziell erhältlich oder können durch Umsetzung von Polyisocyanaten mit geeigneten Blockierungsmitteln nach Bedarf hergestellt werden.

Die einkomponentige härtbare Zusammensetzung kann feuchtigkeitshärtend und / oder hitzehärtend sein.

Unter einer "hitzehärtenden Zusammensetzung" wird im vorliegenden Dokument eine Zusammensetzung mit blockierten Isocyanatgruppen verstanden, bei welcher beim Erhitzen auf eine geeignete Temperatur, typischerweise im Bereich von 120 bis 200 °C, in speziellen Fällen schon bei Temperaturen ab 80 °C, die blockierten Isocyanatgruppen soweit aktiviert werden, dass mit geeigneten Härtern eine Vernetzung und somit Aushärtung eintritt. Dieser Vorgang wird auch als Einbrennen bezeichnet und erfolgt üblicherweise nach der Applikation der Zusammensetzung.

Typischerweise erfolgt die vollständige Aushärtung der beschriebenen einkomponentigen Zusammensetzung durch Einwirkung einer Kombination von Feuchtigkeit und Wärme.

In einer bevorzugten Ausführungsform ist die härtbare Zusammensetzung zweikomponentig.

Als "zweikomponentig" wird im vorliegenden Dokument eine härtbare Zusammensetzung bezeichnet, bei welcher die Bestandteile der Zusammensetzung in zwei verschiedenen Komponenten vorliegen, welche in voneinander getrennten Gebinden gelagert werden und welche jeweils für sich lagerstabil sind. Die beiden Komponenten werden als Komponente **K1** und als Komponente **K2** bezeichnet. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit und / oder Wärme abläuft oder vervollständigt wird.

Besonders bevorzugt sind zweikomponentige härtbare Zusammensetzungen bestehend aus einer Komponente **K1** und einer Komponente **K2,** wobei diese Zusammensetzungen mindestens ein Polyisocyanat **P** und mindestens ein Aldehyd **ALD** der Formel (I) enthalten. Bei deren Aushärtung wirkt der Aldehyd **ALD** aufgrund seiner mindestens zwei Hydroxylgruppen als Härter für das Polyisocyanat **P** und wird unter Kettenverlängerung oder Vernetzung kovalent in das entstehende Polymer eingebaut.

Die Komponente **K1** der besonders bevorzugten härtbaren zweikomponentigen Zusammensetzung enthält mindestens ein Polyisocyanat **P.**

Als Polyisocyanat **P** geeignet ist in einer Ausführungsform ein Polyisocyanat **PI** in der Form eines monomeren Di- oder Triisocyanates oder eines Oligomeren eines monomeren Diisocyanates oder eines Derivates eines monomeren Diisocyanates.

Als monomere Di- oder Triisocyanate geeignet sind beispielsweise die folgenden: 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und - 1,4-diisocyanat, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), α,α,α',α',α",α"-Hexamethyl-1,3,5-mesitylentriisocyanat, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan und Tris-(4-isocyanatophenyl)-thiophosphat.

Als Polyisocyanat **PI** besonders geeignet sind Oligomere oder Derivate von monomeren Diisocyanaten, insbesondere von HDI, IPDI, TDI und MDI. Kommerziell erhältliche Typen sind insbesondere HDI-Biurete, beispielsweise als Desmodur^{®} N 100 und N 3200 (Bayer), Tolonate^{®} HDB und HDB-LV (Rhodia) und Duranate^{®} 24A-100 (Asahi Kasei); HDI-Isocyanurate, beispielsweise als Desmodur^{®} N 3300, N 3600 und N 3790 BA (alle von Bayer), Tolonate^{®} HDT, HDT-LV und HDT-LV2 (Rhodia), Duranate^{®} TPA-100 und THA-100 (Asahi Kasei) und Coronate^{®} HX (Nippon Polyurethane); HDI-Uretdione, beispielsweise als Desmodur^{®} N 3400 (Bayer); HDI-Iminooxadiazindione, beispielsweise als Desmodur^{®} XP 2410 (Bayer); HDI-Allophanate, beispielsweise als Desmodur^{®} VP LS 2102 (Bayer); IPDI-Isocyanurate, beispielsweise in Lösung als Desmodur^{®} Z 4470 (Bayer) oder in fester Form als Vestanat^{®} T1890/100 (Degussa); TDI-Oligomere, beispielsweise als Desmodur^{®} IL (Bayer); sowie gemischte Isocyanurate auf Basis TDI / HDI, zum Beispiel als Desmodur^{®} HL (Bayer). Weiterhin besonders geeeignet sind bei Raumtemperatur flüssige Formen von MDI (sogenanntes "modifiziertes MDI"), welche Gemische von MDI mit MDI-Derivaten, wie beispielsweise MDI-Carbodiimiden bzw. MDI-Uretoniminen oder MDI-Urethanen darstellen, bekannt beispielsweise unter Handelsnamen wie Desmodur^{®} CD, Desmodur^{®} PF, Desmodur^{®} PC (alle von Bayer), sowie Gemische aus MDI und MDI-Homologen (polymeres MDI oder PMDI), erhältlich unter Handelsnamen wie Desmodur^{®} VL, Desmodur^{®} VL50, Desmodur^{®} VL R10, Desmodur^{®} VL R20 und Desmodur^{®} VKS 20F (alle von Bayer), Isonate^{®} M 309, Voranate^{®} M 229 und Voranate^{®} M 580 (alle von Dow) oder Lupranat^{®} M 10 R (von BASF).

Die vorgenannten oligomeren Polyisocyanate **PI** stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid, Uretonimin- oder Oxadiazintrion-Gruppen. Bevorzugt weisen diese Oligomere einen niedrigen Gehalt an monomeren Diisocyanaten auf.

Als Polyisocyanat **PI** bevorzugt sind bei Raumtemperatur flüssige Formen von MDI, sowie die Oligomeren von HDI, IPDI und TDI, insbesondere die Isocyanurate.

Als Polyisocyanat **P** geeignet ist in einer weiteren Ausführungsform ein Isocyanatgruppen aufweisendes Polyurethanpolymer **PUP.**

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Ein geeignetes Isocyanatgruppen aufweisendes Polyurethanpolymer **PUP** ist erhältlich durch die Umsetzung von mindestens einem Polyol mit mindestens einem Polyisocyanat.

Als Polyole für die Herstellung eines Polyurethanpolymers **PUP** können beispielsweise die folgenden Polyole oder Mischungen davon eingesetzt werden:
- Polyetherpolyole, auch Polyoxyalkylenpolyole oder Oligoetherole genannt, welche Polymerisationsprodukte von Ethylenoxid, 1,2-Propylenoxid, 1,2-oder 2,3-Butylenoxid, Tetrahydrofuran oder Mischungen davon sind, eventuell polymerisiert mit Hilfe eines Startermoleküls wie beispielsweise Wasser, Ammoniak, 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.

Als Polyetherpolyole besonders geeignet sind Polyoxyalkylendiole und -triole, insbesondere Polyoxyalkylendiole. Besonders geeignete Polyoxyalkylendi- und triole sind Polyoxyethylendi- und -triole sowie Polyoxypropylendi- und -triole.

Besonders geeignet sind Polyoxypropylendiole und -triole mit einem Ungesättigtheitsgrad tiefer als 0.02 mEq/g und einem Molekulargewicht im Bereich von 1000 bis 30'000 g/mol, sowie Polyoxypropylendiole und -triole mit einem Molekulargewicht von 400 bis 8'000 g/mol. Unter 'Molekulargewicht' oder 'molgewicht' versteht man im vorliegenden Dokument stets das Molekulargewichtsmittel Mₙ. Insbesondere geeignet sind Polyoxypropylendiole mit einem Ungesättigtheitsgrad tiefer als 0.02 mEq/g und einem Molekulargewicht im Bereich von 1000 bis 12'000, insbesondere zwischen 1000 und 8000 g/mol. Solche Polyetherpolyole werden beispielsweise unter dem Handelsnamen Acclaim^{®} von Bayer vertrieben.

Ebenfalls besonders geeignet sind sogenannte "EO-endcapped" (ethylene oxide-endcapped) Polyoxypropylendiole und -triole. Letztere sind spezielle Polyoxypropylenpolyoxyethylenpolyole, die beispielsweise dadurch erhalten werden, dass reine Polyoxypropylenpolyole nach Abschluss der Polypropoxylierung mit Ethylenoxid alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.

Insbesondere geeignet sind Polyesterpolyole, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-caprolacton und Startern wie den vorgenannten zwei- oder dreiwertigen Alkoholen.

Besonders geeignete Polyesterpolyole sind Polyesterdiole.
- Polycarbonatpolyole, wie sie durch Umsetzung beispielsweise der oben genannten - zum Aufbau der Polyesterpolyole eingesetzten - Alkohole mit Dialkylcarbonaten, wie Dimethylcarbonat, Diarylcarbonaten, wie Diphenylcarbonat, oder Phosgen zugänglich sind.
   Besonders geeignet sind Polycarbonatdiole.
- Als Polyole ebenfalls geeignet sind mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylnitril, Vinylchlorid, Vinylacetat, Vinylalkohol, Isobutylen und Isopren, beispielsweise polyhydroxyfunktionelle Acrylnitril/Butadien-Copolymere, welche zum Beispiel aus carboxylterminierten Acrylnitril/Butadien-Copolymeren (kommerziell erhältlich unter dem Namen Hycar^{®} CTBN von Noveon) und Epoxiden oder Aminoalkoholen herstellbar sind; sowie hydrierte polyhydroxyfunktionelle Polymere oder Copolymere von Dienen.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole wie beispielsweise Dimerfettsäurediole, 1,1,1-Trimethylolethan, 1,1,1-Trimethylolpropan, Glycerin, Pentaerythrit, niedrigmolekulare Alkoxylierungsprodukte der vorgenannten zwei- und mehrwertigen Alkohole, sowie Mischungen der vorgenannten Alkohole bei der Herstellung eines Polyurethanpolymers **PUP** mitverwendet werden.

Als Polyisocyanate für die Herstellung eines Polyurethanpolymers **PUP** können aliphatische, cycloaliphatische oder aromatische Polyisocyanate, insbesondere Diisocyanate, verwendet werden, beispielsweise die monomeren Diisocyanate, welche bereits als geeignete Polyisocyanate **PI** erwähnt wurden, sowie Oligomere und Polymere dieser monomeren Diisocyanate, sowie beiliebige Mischungen dieser Isocyanate. Bevorzugt sind monomere Diisocyanate, insbesondere MDI, TDI, HDI und IPDI.

Die Herstellung eines Polyurethanpolymers **PUP** erfolgt in bekannter Art und Weise direkt aus den Polyisocyanaten und den Polyolen, oder durch schrittweise Adduktionsverfahren, wie sie auch als Kettenverlängerungsreaktionen bekannt sind.

In einer bevorzugten Ausführungsform wird das Polyurethanpolymer **PUP** über eine Reaktion von mindestens einem Polyisocyanat und mindestens einem Polyol hergestellt, wobei die Isocyanatgruppen gegenüber den Hydroxylgruppen im stöchiometrischen Überschuss vorliegen. Vorteilhaft beträgt das Verhältnis zwischen Isocyanat- und Hydroxylgruppen 1.3 bis 10, insbesondere 1.5 bis 5.

Vorteilhaft wird die Umsetzung bei einer Temperatur durchgeführt, bei der die verwendeten Polyole, Polyisocyanate und das gebildete Polyurethanpolymer flüssig vorliegen.

Das Polyurethanpolymer **PUP** weist ein Molekulargewicht von vorzugsweise über 500 g/mol, insbesondere ein solches zwischen 1000 und 30'000 g/mol, auf.

Weiterhin weist das Polyurethanpolymer **PUP** bevorzugt eine mittlere NCO-Funktionalität im Bereich von 1.8 bis 3 auf.

Als Polyisocyanat **P** geeignet sind schliesslich auch Gemische enthaltend ein Polyurethanpolymer **PUP** und ein Polyisocyanat **PI,** insbesondere Gemische enthaltend ein MDI-basiertes Polyurethanpolymer **PUP** und monomeres und/oder polymeres MDI, sowie weiterhin Gemische enthaltend ein IPDI-basiertes Polyurethanpolymer **PUP** und monomeres und/oder oligomeres IPDI.

Die Komponente **K2** der besonders bevorzugten härtbaren zweikomponentigen Zusammensetzung enthält mindestens ein Aldehyd **ALD** der Formel (I). Dafür geeignet sind die vorgängig beschriebenen bevorzugen Ausführungsformen, wie sie bereits im Detail beschrieben wurden. Besonders geeignet sind Aldehyde **ALD** der Formel (I) mit Resten R⁴ und R⁵, welche zusammen zwei Hydroxylgruppen aufweisen.

Gegebenenfalls enthält die Komponente **K2** weitere gegenüber Isocyanatgruppen reaktive Verbindungen wie Polyole, Polyamine, Aminoalkohole, Polythiole oder blockierte Amine.

Als Polyole in der Komponente **K2** geeignet sind dieselben Polyole, wie sie bereits als geeignet zur Herstellung eines Polyurethanpolymers **PUP** erwähnt wurden, sowie diejenigen niedrigmolekularen zwei- oder mehrwertigen Alkohole, wie sie vorgängig als geeignet zum Mitverwenden bei der Herstellung eines Polyurethanpolymers **PUP** erwähnt wurden.

Als Polyamine in der Komponente **K2** geeignet sind handelsübliche aliphatische oder aromatische Polyamine mit primären und/ oder sekundären Aminogruppen, wie sie üblicherweise in zweikomponentigen Polyurethanzusammensetzungen eingesetzt werden, wie beispielsweise 1,5-Diamino-2-methylpentan (MPMD), 1,3-Xylylendiamin (MXDA), N,N'-Dibutyl-ethylendiamin, 3,5-Diethyl-2,4(6)-diaminotoluol (DETDA), 3,5-Dimethylthio-2,4(6)-diamino-toluol (Ethacure^{®} 300, Albemarle) sowie primäre und sekundäre Polyoxyalkylen-Diamine, wie sie zum Beispiel unter dem Namen Jeffamine^{®} (von Huntsman Chemicals) erhältlich sind.

Als Aminoalkohole in der Komponente **K2** geeignet sind Verbindungen, welche mindestens eine primäre oder sekundäre Aminogruppe und mindestens eine Hydroxylgruppe aufweisen, wie beispielsweise 2-Aminoethanol, 2-Methylaminoethanol, 1-Amino-2-propanol und Diethanolamin.

Als Polythiole in der Komponente **K2** geeignet sind beispielsweise unter dem Markennamen Thiokol^{®} bekannte flüssige Mercapto-terminierte Polymere, sowie Polyester von Thiocarbonsäuren.

Als gegebenenfalls in der Komponente **K2** vorhandene blockierte Amine geeignet sind beispielsweise Ketimine, Oxazolidine, Enamine und Aldimine. Besonders geeignete blockierte Amine sind Polyaldimine, welche erhältlich sind aus der Umsetzung von Polyaminen mit zwei oder mehr primären Aminogruppen und Aldehyden **ALD** der Formel (I). Weiterhin besonders geeignete blockierte Amine sind Polyaldimine, welche erhältlich sind aus der Umsetzung von Polyaminen mit zwei oder mehr primären Aminogruppen und den Produkten aus der Veresterung von Carbonsäuren, wie sie in WO 2004/013088 A1 beschrieben werden, insbesondere die Produkte aus der Veresterung von Laurinsäure mit 3-Hydroxypivalaldehyd.

In einer Ausführungsform enthält die Komponente **K2** Wasser.

Die besonders bevorzugte härtbare zweikomponentige Zusammensetzung enthält gegebenenfalls weitere Bestandteile, insbesondere in Polyurethanzusammensetzungen üblicherweise eingesetzte Hilfs- und Zusatzstoffe, beispielsweise die folgenden:
- Weichmacher, beispielsweise Carbonsäureester wie Phthalate, beispielsweise Dioctylphthalat, Diisononylphthalat oder Diisodecylphthalat, Adipate, beispielsweise Dioctyladipat, Azelate und Sebacate, organische Phosphor- und Sulfonsäureester oder Polybutene;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-α-Olefine (APAO);
- Lösemittel;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (BaSO₄, auch Schwerspat genannt), Quarzmehle, calcinierte Kaoline, Aluminiumoxide, Aluminiumhydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln;
- Fasern, beispielsweise aus Polyethylen;
- Pigmente, beispielsweise Titandioxid oder Eisenoxide;
- Katalysatoren, welche die Hydrolyse von blockierten Aminogruppen beschleunigen, insbesondere Säuren, beispielsweise organische Carbonsäuren wie Benzoesäure, Salicylsäure oder 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen beschleunigen, beispielsweise Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismut-tris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie beispielsweise Verdickungsmittel oder Thixotropiermittel, zum Beispiel Harnstoffverbindungen, Polyamidwachse, Bentonite oder pyrogene Kieselsäuren;
- Reaktivverdünner und Vernetzer, beispielsweise monomere Diisocyanate sowie Oligomere und Derivate dieser Polyisocyanate, Addukte monomerer Polyisocyanate mit kurzkettigen Polyolen, sowie Adipinsäuredihydrazid und andere Dihydrazide, sowie Polyisocyanate mit blockierten Isocyanatgruppen, wie sie bereits vorgängig erwähnt wurden;
- blockierte Amine, beispielsweise in Form von Ketiminen, Oxazolidinen, Enaminen oder Aldiminen;
- Trocknungsmittel, wie beispielsweise Molekularsiebe, Calciumoxid, hochreaktive Isocyanate wie p-Tosylisocyanat, Orthoameisensäureester, Tetraalkoxysilane wie Tetraethoxysilan;
- Organoalkoxysilane, im Folgenden auch "Silane" genannt, wie beispielsweise Epoxysilane, (Meth)acrylsilane, Isocyanatosilane, Vinylsilane, Carbamatosilane, Alkylsilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, beim Einsatz solcher weiterer Bestandteile darauf zu achten, dass diese die Lagerstabilität der jeweiligen Komponente **K1** oder **K2** der Zusammensetzung nicht stark beeinträchtigen. Falls solche Zusätze als Bestandteil der Komponente **K1** vorhanden sind, ist darauf zu achten, dass diese Zusätze während der Lagerung die Vernetzung der Isocyanatgruppen nicht in signifikantem Ausmass auslösen. Insbesondere bedeutet dies, dass solchermassen verwendete Zusätze kein oder höchstens Spuren von Wasser enthalten sollten. Es kann sinnvoll sein, gewisse Zusätze vor dem Einmischen in die Komponente **K1** chemisch oder physikalisch zu trocknen.

Im Fall der Komponente **K2** sind neben diesen zusätzlich noch weitere Hilfs- und Zusatzstoffe möglich, welche zusammen mit freien Isocyanatgruppen nicht oder nur kurzzeitig lagerfähig sind. Insbesondere sind dies weitere Katalysatoren, insbesondere tertiäre Amine und Metallverbindungen.

Die Herstellung der beiden Komponenten **K1** und **K2** erfolgt getrennt voneinander, für die Komponente **K1** unter Ausschluss von Feuchtigkeit. Die beiden Komponenten **K1** und **K2** sind getrennt voneinander lagerstabil, d.h. sie können jede in einer geeigneten Verpackung oder Anordnung, wie beispielsweise in einem Fass, einem Beutel, einem Eimer, einer Kartusche oder einer Flasche, vor ihrer Anwendung über einen Zeitraum von beispielsweise mehreren Monaten aufbewahrt werden, ohne dass sie sich in ihren jeweiligen Eigenschaften in einem für ihren Gebrauch relevanten Ausmass verändern.

Zur Verwendung der zweikomponentigen Zusammensetzung werden die beiden Komponenten **K1** und **K2** miteinander vermischt. Dabei ist darauf zu achten, dass das Mischungsverhältnis so gewählt wird, dass die gegenüber Isocyanatgruppen reaktiven Bestandteile in einem geeigneten Verhältnis zu den Isocyanatgruppen der Komponente **K1** stehen. Insbesondere beträgt das Verhältnis 0.1 bis 1.1, bevorzugt 0.5 bis 0.95, besonders bevorzugt 0.6 bis 0.9, Equivalent der Summe der vorhandenen Hydroxylgruppen, Aminogruppen, Mercaptogruppen und geschützten Aminogruppen pro Equivalent Isocyanatgruppen, wobei geschützte Aminogruppen in Form von Oxazolidinogruppen doppelt gerechnet werden. Bei der Aushärtung reagieren überschüssige Isocyanatgruppen mit Feuchtigkeit, insbesondere mit Luftfeuchtigkeit.

Das Vermischen der beiden Komponenten **K1** und **K2** erfolgt mit einem geeigneten Verfahren, beispielsweise über einen Statikmischer. Das Vermischen kann kontinuierlich oder batchweise erfolgen. Die vermischte Zusammensetzung wird anschliessend auf ein Substrat appliziert, gegebenenfalls mittels eines geeigneten Applikationshilfsmittels. Dabei muss darauf geachtet werden, dass zwischen dem Vermischen der Komponenten und der Applikation nicht zuviel Zeit vergeht, da durch ein zu starkes Vorreagieren der Bestandteile der vermischten Zusammensetzung vor der Applikation die Funktion der ausgehärteten Zusammensetzung gestört sein kann, beispielsweise indem die Haftung zum Substrat nur ungenügend oder verzögert aufgebaut wird. Die maximale Zeitspanne, innerhalb welcher die vermischte Zusammensetzung appliziert sein sollte, wird als "Topfzeit" bezeichnet.

Nach dem Vermischen der Komponenten **K1** und **K2** beginnt die Aushärtung. Dabei reagieren die gegenüber Isocyanatgruppen reaktiven Gruppen der Komponente **K1** - Hydroxylgruppen, primäre und sekundäre Aminogruppen und Mercaptogruppen - mit den Isocyanatgruppen. Falls die Zusammensetzung blockierte Amine enthält, so reagieren diese ebenfalls mit den Isocyanatgruppen, unter der Voraussetzung, dass ihre blockierten Aminogruppen mit Feuchtigkeit in Kontakt kommen, beispielsweise mit Feuchtigkeit aus der Luft. Überschüssige Isocyanatgruppen reagieren schliesslich direkt mit Wasser, insbesondere mit Feuchtigkeit aus der Luft. Als Ergebnis dieser Reaktionen härtet die Zusammensetzung zu einem festen Material aus; dieser Prozess wird auch als Vernetzung bezeichnet.

Die beschriebene härtbare Zusammensetzung weist eine Reihe von Vorteilen auf.

Ein Aldehyd **ALD** der Formel (I) enthält eine tertiäre Aminogruppe. Derartige Aldehyde können deshalb eine katalytische Wirkung auf die Reaktion der Isocyanatgruppen ausüben und so die Aushärtung beschleunigen. Dabei ist es vorteilhaft, dass seine Basizität vergleichsweise niedrig ist, da stark basische tertiäre Amine die Hydrolyse von gegebenenfalls vorhandenen Aldiminogruppen stören und/oder die direkte Reaktion der Isocyanatgruppen, insbesondere mit Wasser, übermässig beschleunigen können, was eine unvollständige oder unkontrollierte Aushärtung zur Folge haben kann.

Weiterhin wirkt der Aldehyd **ALD** der Formel (I) als Härter für Polyisocyanate, da er an den Resten R⁴ und R⁵ zusammen mindestens zwei Hydroxylgruppen trägt und deshalb mit den Isocyanatgruppen unter Kettenverlängerung oder Vernetzung des entstehenden Polymers reagiert und nicht zu Kettenabbrüchen führt.

Durch den Einbau des Aldehyds **ALD** ins aushärtende Polymer werden auch die potentiell katalytisch wirkenden tertiären Aminogruppen kovalent in das entstehende Polymer eingebaut. Nach dem Einbau ins Polymer ist die katalytische Aktivität der tertiären Aminogruppe aufgrund deren eingeschränkten Beweglichkeit wesentlich reduziert, was für die Beständigkeit des Materials von Vorteil sein kann.

Die Aldehydgruppen bleiben bei der Aushärtung erhalten und werden ebenfalls ins ausgehärtete Material eingebaut. Sie können, falls gewünscht, für weiterführende Reaktionen verwendet werden.

Ein weiterer Vorteil der beschriebenen Zusammensetzungen liegt im vergleichsweise geringen Geruch der Aldehyde **ALD.** Dadurch weisen die Zusammensetzungen vor, während und nach der Aushärtung kaum Geruch auf, was beim Vorhandensein von Aldehyden nicht unbedingt zu erwarten ist.

Bevorzugte Anwendungen der beschriebenen härtbaren Zusammensetzungen sind Klebstoffe, Dichtstoffe, Vergussmassen, Beschichtungen, Bodenbeläge, Anstriche, Lacke, Primer oder Schäume.

Ein weiterer Aspekt der vorliegenden Erfindung betrifft ein Verfahren zum Verkleben von einem Substrat **S1** mit einem Substrat **S2,** welches die Schritte umfasst:
i) Applikation einer vorgängig beschriebenen härtbaren Zusammensetzung auf ein Substrat **S1;**
ii) Kontaktieren der applizierten Zusammensetzung mit einem Substrat **S2** innerhalb der Offenzeit der Zusammensetzung;
oder
i') Applikation einer vorgängig beschriebenen Zusammensetzung auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

Ein weiterer Aspekt betrifft ein Verfahren zum Abdichten. Dieses umfasst den Schritt:
i") Applikation einer vorgängig beschriebenen härtbaren Zusammensetzung zwischen ein Substrat **S1** und ein Substrat **S2,** so dass die Zusammensetzung mit dem Substrat **S1** und dem Substrat **S2** in Kontakt steht;
wobei das Substrat **S2** aus dem gleichen oder einem unterschiedlichen Material wie das Substrat **S1** besteht.

Üblicherweise wird der Dichtstoff in eine sogenannte Fuge eingepresst.

Ein weiterer Aspekt betrifft ein Verfahren zum Beschichten eines Substrates **S1.** Dieses umfasst den Schritt:
i'") Applikation einer vorgängig beschriebenen härtbaren Zusammensetzung auf ein Substrat **S1** innerhalb der Offenzeit der Zusammensetzung.

In diesen drei Verfahren sind geeignete Substrate **S1** und/oder **S2** beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Leder, Stoffe, Papier, Holz, mit Harz gebundene Holzwerkstoffe, Harz-Texil-Compositewerkstoffe, Kunststoffe wie Polyvinylchlorid (Hart- und Weich-PVC), Acrylonitril-Butadien-Styrol-Copolymere (ABS), SMC (Sheet Moulding Composites), Polycarbonat (PC), Polyamid (PA), Polyester, PMMA, Polyester, Epoxidharze, Polyurethane (PUR), Polyoxymethylen (POM), Polyolefine (PO), insbesondere mittels Plasma, Corona oder Flammen oberflächenbehandeltes Polyethylen (PE) oder Polypropylen (PP), Ethylen/Propylen-Copolymere (EPM) und Ethylen/Propylen-Dien-Terpolymere (EPDM); beschichtete Substrate wie pulverbeschichtete Metalle oder Legierungen; sowie Farben und Lacke, insbesondere Automobillacke.

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Die Applikation der härtbaren Zusammensetzung kann in einem breiten Temperaturspektrum erfolgen. Beispielsweise kann die Zusammensetzung bei Raumtemperatur appliziert werden, sie kann aber auch bei tieferen oder bei höheren Temperaturen appliziert werden.

Nach der Applikation der beschriebenen Zusammensetzungen als Klebstoff, Dichtstoff, Vergussmasse, Beschichtung, Bodenbelag, Anstrich, Lack, Primer oder Schaum entsteht ein Artikel. Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie.

### Beispiele

### 1. Beschreibung der Messmethoden

Die **Viskosität** wurde auf einem thermostatisierten Kegel-Platten-Viskosimeter Physica UM (Kegeldurchmesser 20 mm, Kegelwinkel 1 °, Kegelspitze-Platten-Abstand 0.05 mm, Schergeschwindigkeit 10 bis 1000 s⁻¹) gemessen.

Der **Amingehalt,** das heisst der totale Gehalt an freien Aminogruppen und - falls vorhanden - blockierten Aminogruppen (Aldiminogruppen) in den hergestellten Verbindungen, wurde titrimetrisch bestimmt (mit 0.1 N HClO₄ in Eisessig, gegen Kristallviolett) und ist stets angegeben in mmol N/g.

Der *p*Kₐ-Wert für die konjugierte Säure einer Mannich-Base wurde näherungsweise anhand des Halbneutralisations-Potentials bei der potentiometrischen Titration von ca. 1 mmol Mannich-Base in 50 ml Wasser mit 0.1 N HCl bestimmt.

**Infrarotspektren** wurden auf einem FT-IR Gerät 1600 von Perkin-Elmer als unverdünnte Filme auf einer horizontalen ATR-Messeinheit mit ZnSe-Kristall gemessen; die Absorptionsbanden sind in Wellenzahlen (cm⁻¹) angegeben (Messfenster: 4000-650 cm⁻¹), der Zusatz sh weist auf eine als Schulter erscheinende Bande hin, der Zusatz br auf eine breite Bande.

**GC/MS** wurde unter folgenden Bedingungen durchgeführt: Säule Optima-5-MS, 30mx0.25mm, 0.5 µm Filmdicke; Aufheizrate 15 °C/min von 60 °C auf 320 °C, dann 15 min. bei 320 °C gehalten; Trägergas He, 14 psi; Split 15 ml/min; Ionisationsmethode EI⁺. Für das Gaschromatogramm ist die Retentionszeit des Produktsignals (t_{R}) angegeben. Beim Massenspektrum werden nur die grössten Peaks angegeben (als m/z); die relative Intensität (in %) und, wenn möglich, eine tentative Zuordnung des Molekülfragments stehen in Klammern.

### 2. Herstellung von Aldehyden

### Beispiel 1: Aldehyd ALD-1

### 3-(N-Bis(2-hydroxyethyl)amino)-2,2-dimethyl-propanal:

In einem Rundkolben wurden unter Stickstoffatmosphäre 83.4 g (1.00 mol) 36%iger wässriger Formaldehyd und 75.7 g (1.05 mol) Isobutyraldehyd vorgelegt. Unter gutem Rühren und Eiskühlung wurden aus einem Eintropftrichter langsam 105.1 g (1.00 mol) Diethanolamin zugetropft, wobei darauf geachtet wurde, dass die Temperatur der Reaktionsmischung nicht über 20 °C anstieg. Nach erfolgter Zugabe liess man eine Stunde bei Raumtemperatur rühren. Die entstandene klare, farblose Reaktionsmischung wurde im Ölbad bei 80 °C während 2 Stunden unter Rückfluss gerührt, auf Raumtemperatur abgekühlt und die flüchtigen Bestandteile im Wasserstrahlvakuum bei 80 °C abdestilliert. Man erhielt 181.2 g (96% der Theorie) Rohprodukt als klares, gelbliches Öl, das einen Amingehalt von 5.40 mmol N/g und eine Viskosität von 23.7 Pa·s bei 20 °C aufwies. Das Rohprodukt enthielt neben 3-(N-Bis(2-hydroxyethyl)amino)-2,2-dimethyl-propanal kleinere Anteile von 3-Hydroxy-2,2-dimethyl-propanal, N-(2-Hydroxyethyl)-oxazolidin und N-(2-Hydroxyethyl)-2-isopropyl-oxazolidin (nach GC/MS-Untersuchung).
*p*Kₐ ≈ 7.1.
IR: 3358br (OH), 2950, 2929sh, 2913, 2870, 2830, 2719sh br (CHO), 1721 (C=O),1464,1391,1359,1302br,1206,1147,1078sh,1037,966,940,920,883, 786. GC/MS: t_{R} = 10.3 min; Massenspektrum: 189 (2, [M]⁺), 172 (3, [M-OH]⁺), 158 (11, [M-CH₂OH]⁺), 128 (4), 118 (100, [M-C(CH₃)₂CHO]⁺), 116 (15), 102 (6), 98 (5), 88 (2, [118-CHOH]⁺), 88 (72), 86 (21), 74 (50), 56 (51).

### Beispiel 2: Aldehyd ALD-2

### 3-(N-Bis(2-hydroxy-2-methylethyl)amino)-2,2-dimethyl-propanal:

Unter gleichen Bedingungen wie in Beispiel 1 beschrieben wurden 83.4 g (1.00 mol) 36%iger wässriger Formaldehyd mit 75.7 g (1.05 mol) Isobutyraldehyd und 133.2 g (1.00 mol) Diisopropanolamin umgesetzt und aufgearbeitet. Man erhielt 199.4 g (92% der Theorie) Rohprodukt als klares, gelbliches Öl, das einen Amingehalt von 4.87 mmol N/g und eine Viskosität von 8.2 Pa·s bei 20 °C aufwies. Das Rohprodukt enthielt neben 3-(N-Bis(2-hydroxy-2-methylethyl)amino)-2,2-dimethyl-propanal kleinere Anteile von 3-Hydroxy-2,2-dimethyl-propanal, N-(2-Hydroxy-2-methylethyl)-oxazolidin und N-(2-Hydroxy-2-methylethyl)-2-isopropyl-oxazolidin (nach GC/MS-Untersuchung).
*p*Kₐ ≈ 7.1.
IR: 3392br (OH), 2966, 2933, 2872, 2818, 2719sh br (CHO), 1722 (C=O), 1461, 1409, 1375, 1328, 1274, 1209, 1158, 1130, 1090sh, 1055, 1028sh, 978, 945, 914, 891, 864, 839, 818, 786.
GC/MS: t_{R} = 10.3 min; Massenspektrum: 217 (3, [M]⁺), 172 (30, [M-CH(CH₃)OH]⁺), 146 (44, [M-C(CH₃)₂CHO]⁺), 144 (21), 130 (6), 126 (6), 116 (7), 114 (10), 102 (100, [146-C(CH₃)OH]⁺), 100 (18), 88 (16), 70 (38).

### Beispiel 3: Aldehyd ALD-3

### 3-(1-(4-(2-hydroxyethyl)piperazino))-2,2-dimethyl-propanal:

Unter gleichen Bedingungen wie in Beispiel 1 beschrieben wurden 32.0 g (0.38 mol) 36%iger wässriger Formaldehyd mit 29.1 g (0.40 mol) Isobutyraldehyd und 50.0 g (0.38 mol) 1-(2-Hydroxyethyl)-piperazin umgesetzt und aufgearbeitet. Man erhielt 81.4 g (99% der Theorie) Rohprodukt als klares, dickflüssiges, orangefarbenes Öl, das einen Amingehalt von 9.17 mmol N/g aufwies.
IR: 3395br (OH), 3170br, 2944, 2869, 2806, 2768sh, 2695 (CHO), 1723 (C=O), 1458, 1402sh, 1374, 1361sh, 1349, 1336, 1318, 1294, 1276, 1153, 1120, 1096, 1085sh, 1052, 1011, 974sh, 942, 913, 877, 834, 815, 771.

### 3. Verwendung von Aldehyden als Härter in Polyurethanzusammensetzungen

### Beispiele 4 und 5 und Vergleichsbeispiele 6 und 7: 2K-Vergussmassen

Für jedes Beispiel wurden die jeweiligen Bestandteile der Komponente ***K2*** gemäss Tabelle 1 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) zu einer homogenen Creme gemischt. Dazu wurden die in der Tabelle 1 angegebenen Gewichtsteile an PMDI als Komponente ***K1*** zugegeben und eingemischt (30 sec. bei 3000 U/min). Das Verhältnis zwischen den Isocyanatgruppen der Komponente ***K1*** und den Hydroxylgruppen der Komponente ***K2*** beträgt stets 1.1.

**Tabelle 1: Zusammensetzung der zweikomponentigen Vergussmassen.**

| Beispiel | **4** | **5** | **6 (Vgl.)** | **7 (Vgl.)** |
|---|---|---|---|---|
| **Komponente *K1***: | | | | |
| PMDI^{a} | 38.0 | 37.2 | 37.7 | 29.7 |
| **Komponente *K2*:** | | | | |
| Ricinusöl^{b} | 22.5 | 22.5 | 22.5 | 22.5 |
| Dimerfettsäurediol^{c} | 17.5 | 17.5 | 17.5 | 22.5 |
| Triol^{d} | 4.75 | 4.75 | 4.75 | 4.75 |
| Diol^{e} | ***ALD-1,*** 5.0 | ***ALD-2,*** 5.0 | ***TPG*,** 5.0 | - |
| Säurekatalysator^{f} | 0.25 | 0.25 | 0.25 | 0.25 |
| Molekularsieb^{g} | 2.0 | 2.0 | 2.0 | 2.0 |
| Kreide ^{h} | 48.0 | 48.0 | 48.0 | 48.0 |

| | | | | |
|---|---|---|---|---|
| ^{a} Desmodur^{®} VKS 20 F, Bayer; NCO-Gehalt = 30.0 Gew.-%. ^{b} Fluka; OH-Zahl = 165 mg KOH/g. ^{c} Sovermol^{®} 908, Cognis;OH-Zahl = 200 mg KOH/g. ^{d} Desmophen^{®} 4011 T, Bayer; OH-Zahl = 550 mg KOH/g. ^{e} als Diole verwendet wurden die Aldehyde ***ALD-1*** aus Beispiel 1 und ***ALD-2*** aus Beispiel 2 und ***TPG*** = Tripropylenglykol, OH-Zahl = 583 mg KOH/g. ^{f} Salicylsäure (5 Gew.-% in Dioctyladipat). ^{g} Purmol^{®} 4ST, CU Chemie Uetikon; Porengrösse 4 A. ^{h} Omyacarb^{®} 5-GU, Omya. | | | | |

Die so erhaltenen Vergussmassen wurden auf Aushärtegeschwindigkeit und mechanische Eigenschaften geprüft. Hinweise zur Aushärtegeschwindigkeit wurden zum einen durch Messen der **Zeit bis zur Klebefreiheit** (tack-free time) der Vergussmasse, unmittelbar nach dem Mischen der Komponenten ***K1*** und ***K2**,* erhalten. Dazu wurde die vermischte Zusammensetzung in einer Schichtdicke von ca. 2 mm auf Pappkarton aufgetragen und im Normklima (23±1 °C, 50±5% relative Luftfeuchtigkeit) die Zeit bestimmt, die es dauerte, bis beim leichten Antippen der Oberfläche der Beschichtungsmasse mittels einer Pipette aus LDPE erstmals keine Rückstände auf der Pipette mehr zurückblieben. Zum anderen wurde der weitere Verlauf der Aushärtung durch periodisches Messen der **Shore D-Härte** nach DIN EN 53505 verfolgt.

Zur Prüfung der mechanischen Eigenschaften wurde die Vergussmasse als Film mit einer Schichtdicke von ca. 2 mm in eine plane PTFE-Form gegossen, der Film während 7 Tagen im Normklima ausgehärtet und nach DIN EN 53504 auf **Zugfestigkeit, Bruchdehnung** und **E-Modul** (bei 0.5 - 3.0 % Dehnung, Zuggeschwindigkeit: 10 mm/min) geprüft.

Die Ergebnisse dieser Prüfungen sind in Tabelle 2 aufgeführt.

**Tabelle 2: Eigenschaften der zweikomponentigen Vergussmassen.**

| Beispiel | **4** | **5** | **6 (Vgl.)** | **7 (Vgl.)** |
|---|---|---|---|---|
| Klebefreiheit (min.)^{a} | 34 | 27 | 108 | 48 |
| Shore D nach 1 Tag | 83 | 89 | 71 | 60 |
| Shore D nach 3 Tagen | 93 | 94 | 84 | 75 |
| Shore D nach 7 Tagen | 95 | 96 | 85 | 82 |
| Shore D getempert^{b} | 96 | 96 | 94 | 88 |
| Zugfestigkeit (MPa) | 14.6 | 30.4 | 9.6 | 8.1 |
| Bruchdehnung (%) | 6 | 3.5 | 30 | 60 |
| E-Modul (MPa) | 370 | 770 | 175 | 100 |

| | | | | |
|---|---|---|---|---|
| ^{a} Zeit bis zur Klebefreiheit (tack-free time) in Minuten. ^{b} 4 h bei 105°C der während 7 Tagen im Normklima ausgehärteten Prüfkörper. | | | | |

### 4. Verwendung von Aldehyden zur Herstellung von Aldiminen

### Beispiel 8: Dialdimin ausgehend von Aldehyd ALD-1

In einem Rundkolben wurden unter Stickstoffatmosphäre 68.2 g Polyetherdiamin (Polyoxypropylen-Diamin mit einem mittleren Molekulargewicht von ca. 240 g/mol; Jeffamine^{®} D-230, Huntsman; Amingehalt 8.29 mmol N/g) und 117.6 g Aldehyd ***ALD-1*** aus Beispiel 1 eingewogen und die Mischung während einer Stunde bei Raumtemperatur gerührt. Danach wurden die flüchtigen Bestandteile im Vakuum entfernt (10 mbar, 80 °C). Ausbeute: 177.1 g eines klaren, gelben Öls mit einem Amingehalt von 6.78 mmol N/g und einer Viskosität von 9.8 Pa·s bei 20 °C.
IR: 3391 br (OH), 2964, 2926, 2868, 1662 (C=N), 1469, 1456sh, 1392sh, 1373, 1294, 1106sh, 1049, 1004sh, 926, 903, 877.

### Beispiel 9: Dialdimin ausgehend von Aldehyd ALD-2

Unter gleichen Bedingungen wie in Beispiel 8 beschrieben wurden 27.2 g Isophorondiamin (Vestamin^{®} IPD, Degussa; Amingehalt 11.67 mmol N/g) und 71.8 g Aldehyd ***ALD-2*** aus Beispiel 2 umgesetzt. Ausbeute: 93.2 g eines klaren, gelben Honigs mit einem Amingehalt von 7.66 mmol N/g und einer Viskosität von 150 Pa·s bei 20 °C.
IR: 3393br (OH), 2962, 2926, 2898, 2868, 2837, 2818, 1662 (C=N), 1459, 1408, 1373, 1364, 1333, 1273, 1159, 1133, 1116sh, 1058, 1003, 976sh, 945, 909, 891sh, 864, 838.

### Beispiel 10: Dialdimin ausgehend von Aldehyd ALD-2

Unter gleichen Bedingungen wie in Beispiel 8 beschrieben wurden 37.7 g Polyetherdiamin (Polyoxypropylen-Diamin mit einem mittleren Molekulargewicht von ca. 240 g/mol; Jeffamine^{®} D-230, Huntsman; Amingehalt 8.29 mmol N/g) und 70.6 g Aldehyd ***ALD-2*** aus Beispiel 2 umgesetzt. Ausbeute: 103.4 g eines klaren, gelb-bräunlichen Öls mit einem Amingehalt von 6.26 mmol N/g und einer Viskosität von 4.0 Pa·s bei 20 °C.
IR: 3419br (OH), 2965, 2925, 2918, 2868, 2822sh, 1662 (C=N), 1457, 1408sh, 1373,1331,1274,1196sh,1106,1089,1059,1019, 1002, 977, 944, 910, 865, 838.

### Beispiel 11: Monoaldimin ausgehend von Aldehyd ALD-1

Unter gleichen Bedingungen wie in Beispiel 8 beschrieben wurden 6.55 g 2-(2-Aminoethoxy)-ethanol (DGA; Diglycolamine^{®} Agent, Huntsman; Amingehalt 9.39 mmol N/g) und 13.36 g Aldehyd ***ALD-1*** aus Beispiel 1 umgesetzt. Ausbeute: 16.25 g eines klaren, gelben Öls mit einem Amingehalt von 7.18 mmol N/g und einer Viskosität von 3.4 Pa·s bei 20 °C.
IR: 3358br (OH), 2928, 2865, 2716sh, 1943br, 1663 (C=N), 1467, 1459, 1391, 1358, 1285, 1238, 1123, 1044, 1003sh, 940sh, 924sh, 890, 815, 785, 770.

## Patentansprüche

1. Aldehyd **ALD** der Formel (I) 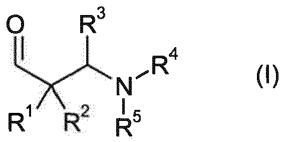 wobei
R¹ und R² entweder
unabhängig voneinander jeweils für einen einwertigen Kohlenwasserstoffrest mit 1 bis 12 C-Atomen stehen
oder
zusammen für einen zweiwertigen Kohlenwasserstoffrest mit 4 bis 12 C-Atomen, der Teil eines, gegebenenfalls substituierten, carbocyclischen Rings mit 5 bis 8 C-Atomen ist, stehen;
R³ für ein Wasserstoffatom oder eine Alkylgruppe oder eine Arylalkylgruppe oder eine Alkoxycarbonylgruppe steht;
R⁴ und R⁵ entweder
unabhängig voneinander jeweils für eine Methylgruppe oder einen einwertigen aliphatischen, cycloaliphatischen oder arylaliphatischen Rest mit 2 bis 20 C-Atomen stehen, welcher gegebenenfalls Hydroxylgruppen oder Aldehydgruppen aufweist, und welcher gegebenenfalls Heteroatome in der Form von Ether-Sauerstoff oder tertiärem Amin-Stickstoff enthält, mit der Massgabe, dass R⁴ mindestens eine Hydroxylgruppe aufweist und dass R⁴ und R⁵ zusammen mindestens zwei Hydroxylgruppen aufweisen,
oder
zusammen für einen mindestens zwei Hydroxylgruppen aufweisenden zweiwertigen aliphatischen Rest mit 4 bis 20 C-Atomen, der Teil eines, gegebenenfalls substituierten, heterocyclischen Rings mit 5 bis 8 Ringatomen ist, wobei dieser Ring gegebenenfalls weitere Heteroatome in der Form von Ether-Sauerstoff oder tertiärem Amin-Stickstoff enthält und gegebenenfalls Aldehydgruppen aufweist, stehen.

2. Aldehyd gemäss Anspruch 1, **dadurch gekennzeichnet, dass** R⁴ und R⁵ jeweils für eine 2-Hydroxyethylgruppe oder für eine 2-Hydroxypropylgruppe stehen.

3. Aldehyd gemäss Anspruch 1, **dadurch gekennzeichnet, dass** R⁴ zwei Hydroxylgruppen aufweist und dass R⁵ keine Hydroxylgruppe aufweist.

4. Aldehyd gemäss Anspruch 1, **dadurch gekennzeichnet, dass** der Aldehyd **ALD** ein Aldehyd der Formel (VI) ist.

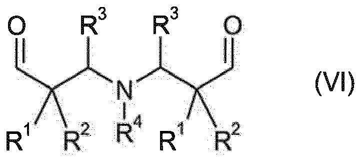

5. Aldehyd der Formel (V) 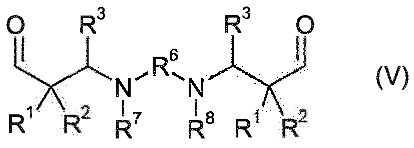 wobei
R¹, R² und R³ die in Anspruch 1 genannte Bedeutung aufweisen,
R⁶ für einen zweiwertigen aliphatischen, cycloaliphatischen oder arylaliphatischen Rest mit 2 bis 20 C-Atomen, welcher gegebenenfalls Hydroxylgruppen aufweist, und welcher gegebenenfalls Heteroatome in der Form von Ether-Sauerstoff oder tertiärem Amin-Stickstoff enthält, steht;
R⁷ und R⁸ entweder
unabhängig voneinander jeweils für eine Methylgruppe oder einen einwertigen aliphatischen, cycloaliphatischen oder arylaliphatischen Rest mit 2 bis 20 C-Atomen, welcher gegebenenfalls Hydroxylgruppen aufweist, und welcher gegebenenfalls Heteroatome in der Form von Ether-Sauerstoff oder tertiärem Amin-Stickstoff enthält, stehen;
oder
zusammen für einen mindestens eine Hydroxylgruppe aufweisenden zweiwertigen aliphatischen Rest mit 4 bis 20 C-Atomen stehen, welcher zusammen mit N-R⁶-N einen heterocyclischen Ring mit 5 bis 8 Ring-Atomen bildet, wobei dieser Ring gegebenenfalls weitere Heteroatome in der Form von Ether-Sauerstoff oder tertiärem Amin-Stickstoff enthält,
mit der Massgabe, dass mindestens einer der Reste R⁶, R⁷ und R⁸ mindestens eine Hydroxylgruppe aufweist.

6. Aldehyd der Formel (VII), 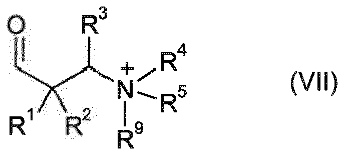 wobei
R¹, R², R³, R⁴ und R⁵ die in Anspruch 1 genannte Bedeutung aufweisen, und
R⁹ für ein Wasserstoffatom oder für einen Alkyl-, Cycloalkyl- oder Arylalkylrest mit 1 bis 20 C-Atomen steht, und der Aldehyd der Formel (VII) durch Protonierung oder Alkylierung aus einem Aldehyd **ALD** der Formel (I) gemäss einem der Ansprüche 1 bis 5 erhältlich ist.

7. Verfahren zur Herstellung eines Aldehyds gemäss einem der Ansprüche 1 bis 3, **dadurch gekennzeichnet, dass** ein Aldehyd **Y1** der Formel (II), ein Aldehyd Y2 der Formel (III) und ein sekundäres aliphatisches, mindestens zwei Hydroxylgruppen aufweisendes Amin **C** der Formel (IV) unter Wasserabspaltung zu einem Aldehyd **ALD** der Formel (I) umgesetzt werden, 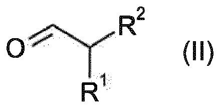 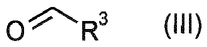 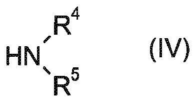 wobei
R¹, R², R³, R⁴ und R⁵ die in Anspruch 1 genannte Bedeutung aufweisen.

8. Verfahren zur Herstellung eines Aldehyds gemäss Anspruch 5, **dadurch gekennzeichnet, dass** ein Aldehyd **Y1** der Formel (II), ein Aldehyd **Y2** der Formel (III) und ein sekundäres aliphatisches, mindestens eine Hydroxylgruppe aufweisendes Amin **C** der Formel (IV) unter Wasserabspaltung zu einem Aldehyd **ALD** der Formel (V) umgesetzt werden, 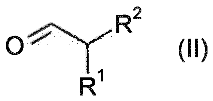 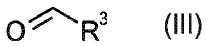 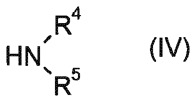 wobei
R¹, R², R³, R⁴ und R⁵ die in Anspruch 1 genannte Bedeutung aufweisen.

9. Verfahren zur Herstellung eines Aldehyds gemäss Anspruch 4, **dadurch gekennzeichnet, dass** mindestens ein primäres aliphatisches, mindestens eine Hydroxylgruppe aufweisendes Amin **C'** der Formel (IV') mit einem Aldehyd **Y1** der Formel (II) und einem Aldehyd **Y2** der Formel (III) im Molverhältnis 1:2:2 unter Wasserabspaltung zu einem Aldehyd der Formel (VI) umgesetzt wird. 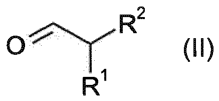 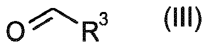
H₂N-R⁴ (IV')
wobei
R¹, R², R³ und R⁴ die in Anspruch 1 genannte Bedeutung aufweisen.

10. Verwendung eines Aldehyds **ALD** der Formel (I) gemäss einem der Ansprüche 1 bis 4, eines Aldehyds der Formel (V) gemäss Anspruch 5 oder eines Aldehyds der Formel (VII) gemäss Anspruch 6 als Bestandteil von Zusammensetzungen basierend auf Isocyanaten oder Epoxidharzen.

11. Mindestens eine Aldehydgruppe aufweisendes Polyol, welches erhältlich ist aus der Polyalkoxylierung eines Aldehyds **ALD** der Formel (I) gemäss einem der Ansprüche 1 bis 4 oder eines Aldehyds der Formel (V) gemäss Anspruch 5.

12. Härtbare Zusammensetzung enthaltend mindestens ein Polyisocyanat und mindestens ein Aldehyd **ALD** der Formel (I) gemäss einem der Ansprüche 1 bis 4, ein Aldehyd der Formel (V) gemäss Anspruch 5 oder ein Aldehyd der Formel (VII) gemäss Anspruch 6.

13. Ausgehärtete Zusammensetzung erhalten durch die Reaktion einer härtbaren Zusammensetzung gemäss Anspruch 12 und Wasser.

14. Verfahren zum Verkleben von einem Substrat **S1** mit einem Substrat **S2,** welches die Schritte umfasst
i) Applikation einer härtbaren Zusammensetzung gemäss Anspruch 12 auf ein Substrat **S1;**
ii) Kontaktieren der applizierten Zusammensetzung mit einem Substrat **S2** innerhalb der Offenzeit der Zusammensetzung
oder
i') Applikation einer Zusammensetzung gemäss Anspruch 12 auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

15. Artikel, welcher nach einem Verfahren gemäss Anspruch 14 verklebt wurde.

## Claims

1. Aldehyde **ALD** of the formula (I) 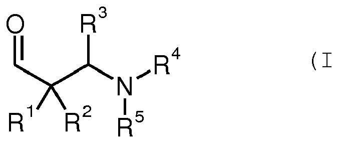 where
R¹ and R² are either each independently a monovalent hydrocarbon radical having 1 to 12 carbon atoms
or
together are a divalent hydrocarbon radical having 4 to 12 carbon atoms which is part of an optionally substituted carbocyclic ring having 5 to 8 carbon atoms;
R³ is a hydrogen atom or an alkyl group or an arylalkyl group or an alkoxycarbonyl group;
R⁴ and R⁵ are either
each independently a methyl group or a monovalent aliphatic, cycloaliphatic or arylaliphatic radical which has 2 to 20 carbon atoms and optionally has hydroxyl groups or aldehyde groups and optionally contains heteroatoms in the form of ether oxygen or tertiary amine nitrogen, with the proviso that R⁴ has at least one hydroxyl group and that R⁴ and R⁵ together have at least two hydroxyl groups,
or
together are a divalent aliphatic radical which has at least two hydroxyl groups and 4 to 20 carbon atoms, and is part of an optionally substituted heterocyclic ring having 5 to 8 ring atoms, this ring optionally containing further heteroatoms in the form of ether oxygen or tertiary amine nitrogen, and optionally having aldehyde groups.

2. Aldehyde according to Claim 1, **characterized in that** R⁴ and R⁵ are each a 2-hydroxyethyl group or a 2-hydroxypropyl group.

3. Aldehyde according to Claim 1, **characterized in that** R⁴ has a hydrogen atom and that R⁵ has no hydroxyl group.

4. Aldehyde according to Claim 1, **characterized in that** the aldehyde ALD is an aldehyde of the formula (VI)

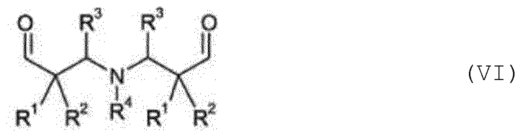

5. Aldehyde of the formula (V) 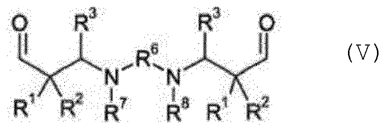 where
R¹, R² and R³ have the meaning mentioned in Claim 1,
R⁶ is a divalent aliphatic, cycloaliphatic or arylaliphatic radical with 2 to 20 carbon atoms, optionally having hydroxyl groups and optionally containing heteroatoms in the form of ether oxygen or tertiary amine nitrogen;
R⁷ and R⁸ are either
each independently a methyl group or a monovalent aliphatic, cycloaliphatic or arylaliphatic radical which has 2 to 20 carbon atoms and optionally has hydroxyl groups, and which optionally contains heteroatoms in the form of ether oxygen or tertiary amine nitrogen,
or
together are a divalent aliphatic radical which has at least one hydroxyl group and 4 to 20 carbon atoms, and together with N-R⁶-N forms a heterocyclic ring having 5 to 8 ring atoms, this ring optionally containing further heteroatoms in the form of ether oxygen or tertiary amine nitrogen,
with the proviso that at least one of the radicals R⁶, R⁷ and R⁸ has at least one hydroxyl group.

6. Aldehyde of the formula (VII), 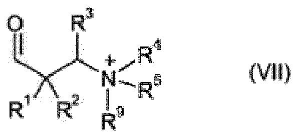 where
R¹, R², R³, R⁴ and R⁵ have the meaning mentioned in Claim 1
and
R⁹ is a hydrogen atom or an alkyl, cycloalkyl or arylalkyl radical with 1 to 20 carbon atoms and the aldehyde of the formula (VII) can be obtained by protonation or alkylation from an aldehyde **ALD** of the formula (I) according to any one of claims 1 to 5.

7. Process for preparing an aldehyde according to any of claims 1 to 3, **characterized in that** an aldehyde **Y1** of the formula (II), an aldehyde **Y2** of the formula (III) and a secondary aliphatic amine **C** of the formula (IV) having at least two hydroxyl groups are reacted, splitting off water, to form an aldehyde **ALD** of the formula (I) 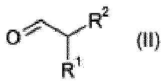 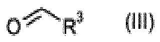 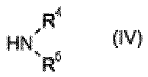 where
R¹, R², R³, R⁴ and R⁵ have the meaning mentioned in Claim 1.

8. Process for preparing an aldehyde according to claim 5, **characterized in that** an aldehyde **Y1** of the formula (II), an aldehyde **Y2** of the formula (III) and a secondary aliphatic amine **C** of the formula (IV) having at least one hydroxyl group are reacted, splitting off water, to form an aldehyde **ALD** of the formula (V) 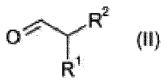 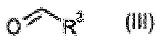 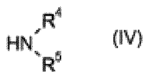 where
R¹, R², R³, R⁴ and R⁵ have the meaning mentioned in Claim 1.

9. Process for preparing an aldehyde according to claim 4, **characterized in that** at least one primary aliphatic amine **C'** of the formula (IV') having at least one hydroxyl group is reacted with an aldehyde **Y1** of the formula (II) and an aldehyde **Y2** of the formula (III) in a molar ratio of 1:2:2, splitting off water, to form an aldehyde of the formula (VI) 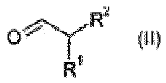 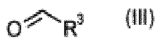 H₂N-R⁴ (IV') where
R¹, R², R³ and R⁴ have the meaning mentioned in Claim 1.

10. Use of an aldehyde **ALD** of the formula (I) according to any of Claims 1 to 4, of an aldehyde of the formula (V) according to Claim 5 or of an aldehyde of the formula (VII) according to Claim 6 as a constituent of compositions based on isocyanates or epoxy resins.

11. Polyol which has at least one aldehyde group and is obtainable from the polyalkoxylation of an aldehyde **ALD** of the formula (I) according to any of Claims 1 to 4 or an aldehyde of the formula (V) according to Claim 5.

12. Curable composition containing at least one polyisocyanate and at least one aldehyde **ALD** of the formula (I) according to any of Claims 1 to 4, an aldehyde of the formula (V) according to Claim 5 or an aldehyde of the formula (VII) according to Claim 6.

13. Cured composition obtained by reacting a curable composition according to Claim 12 and water.

14. Process for bonding a substrate **S1** to a substrate **S2,** which comprises the steps of
i) applying a curable composition according Claim 12 to a substrate **S1;**
ii) contacting the applied composition with a substrate **S2** within the open time of the composition;
or
i') applying a composition according to Claim 12 to a substrate **S1** and to a substrate **S2;**
ii') contacting the applied compositions with one another within the open time of the composition;
the substrate **S2** consisting of the same material as, or a different material than the substrate **S1.**

15. Article which has been bonded by a process according to Claim 14.

## Revendications

1. Aldéhyde **ALD** de formule (I) 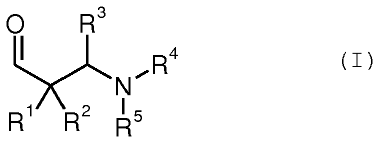 où
R¹ et R² sont soit
chacun indépendamment un radical hydrocarbure monovalent comportant 1 à 12 atomes de carbone
soit
sont ensemble un radical hydrocarbure divalent comportant 4 à 12 atomes de carbone qui fait partie d'un cycle carbocyclique facultativement substitué comportant 5 à 8 atomes de carbone ;
R³ est un atome d'hydrogène ou un groupe alkyle ou un groupe arylalkyle ou un groupe alcoxycarbonyle ;
R⁴ et R⁵ sont soit
chacun indépendamment un groupe méthyle ou un radical aliphatique, cycloaliphatique ou arylaliphatique monovalent qui comporte 2 à 20 atomes de carbone et comporte facultativement des groupes hydroxyle ou des groupes aldéhyde et contient facultativement des hétéroatomes sous la forme d'oxygène d'éther ou d'azote d'amine tertiaire, à condition que R⁴ comporte au moins un groupe hydroxyle et que R⁴ et R⁵ comportent ensemble au moins deux groupes hydroxyle,
soit
sont ensemble un radical aliphatique divalent qui comporte au moins deux groupes hydroxyle et 4 à 20 atomes de carbone, et fait partie d'un cycle hétérocyclique facultativement substitué comportant 5 à 8 atomes de cycle, ce cycle contenant facultativement des hétéroatomes supplémentaires sous la forme d'oxygène d'éther ou d'azote d'amine tertiaire, et comportant facultativement des groupes aldéhyde.

2. Aldéhyde selon la revendication 1, **caractérisé en ce que** R⁴ et R⁵ sont chacun un groupe 2-hydroxyéthyle ou un groupe 2-hydroxypropyle.

3. Aldéhyde selon la revendication 1, **caractérisé en ce que** R⁴ comporte un atome d'hydrogène et **en ce que** R⁵ ne comporte pas de groupe hydroxyle.

4. Aldéhyde selon la revendication 1, **caractérisé en ce que** l'aldéhyde ALD est un aldéhyde de formule (VI)

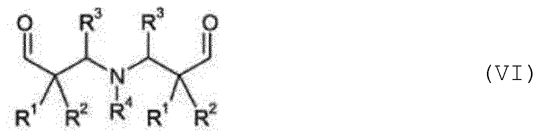

5. Aldéhyde de formule (V) 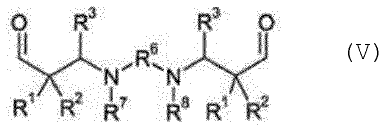 où
R¹, R² et R³ ont la signification mentionnée dans la revendication 1,
R⁶ est un radical aliphatique, cycloaliphatique ou arylaliphatique divalent avec 2 à 20 atomes de carbone, comportant facultativement des groupes hydroxyle et contenant facultativement des hétéroatomes sous la forme d'oxygène d'éther ou d'azote d'amine tertiaire ;
R⁷ et R⁸ sont soit
chacun indépendamment un groupe méthyle ou un radical aliphatique, cycloaliphatique ou arylaliphatique monovalent qui comporte 2 à 20 atomes de carbone et comporte facultativement des groupes hydroxyle, et qui contient facultativement des hétéroatomes sous la forme d'oxygène d'éther ou d'azote d'amine tertiaire,
soit
sont ensemble un radical aliphatique divalent qui comporte au moins un groupe hydroxyle et 4 à 20 atomes de carbone, et conjointement avec N-R⁶-N forme un cycle hétérocyclique comportant 5 à 8 atomes de cycle, ce cycle contenant facultativement des hétéroatomes supplémentaires sous la forme d'oxygène d'éther ou d'azote d'amine tertiaire,
à condition qu'au moins l'un des radicaux R⁶, R⁷ et R⁸ comporte au moins un groupe hydroxyle.

6. Aldéhyde de formule (VII), 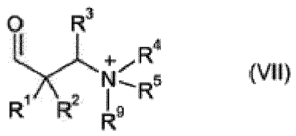 où
R¹, R², R³, R⁴ et R⁵ ont la signification mentionnée dans la revendication 1
et
R⁹ est un atome d'hydrogène ou un radical alkyle, cycloalkyle ou arylalkyle avec 1 à 20 atomes de carbone et l'aldéhyde de formule (VII) peut être obtenu par protonation ou alkylation à partir d'un aldéhyde **ALD** de formule (I) selon l'une quelconque des revendications 1 à 5.

7. Procédé de préparation d'un aldéhyde selon l'une quelconque des revendications 1 à 3, **caractérisé en ce qu'**un aldéhyde **Y1** de formule (II), un aldéhyde **Y2** de formule (III) et une amine aliphatique secondaire **C** de formule (IV) comportant au moins deux groupes hydroxyle sont mis à réagir, en enlevant l'eau, pour former un aldéhyde **ALD** de formule (I) 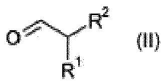 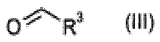 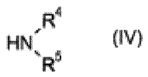 où
R¹, R², R³, R⁴ et R⁵ ont la signification mentionnée dans la revendication 1.

8. Procédé de préparation d'un aldéhyde selon la revendication 5, **caractérisé en ce qu'**un aldéhyde **Y1** de formule (II), un aldéhyde **Y2** de formule (III) et une amine aliphatique secondaire **C** de formule (IV) comportant au moins un groupe hydroxyle sont mis à réagir, en enlevant l'eau, pour former un aldéhyde **ALD** de formule (V)

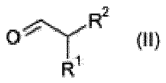

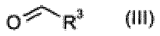

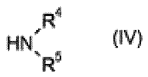

9. Procédé de préparation d'un aldéhyde selon la revendication 4, **caractérisé en ce qu'**au moins une amine aliphatique primaire **C'** de formule (IV') comportant au moins un groupe hydroxyle est mise à réagir avec un aldéhyde **Y1** de formule (II) et un aldéhyde **Y2** de formule (III) dans un rapport molaire de 1 : 2 : 2, en enlevant l'eau, pour former un aldéhyde de formule (VI) 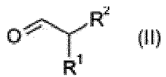 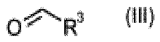
H₂N-R⁴ (IV')
où
R¹, R², R³ et R⁴ ont la signification mentionnée dans la revendication 1.

10. Utilisation d'un aldéhyde **ALD** de formule (I) selon l'une quelconque des revendications 1 à 4, d'un aldéhyde de formule (V) selon la revendication 5 ou d'un aldéhyde de formule (VII) selon la revendication 6 comme constituant de compositions à base d'isocyanates ou de résines époxy.

11. Polyol qui comporte au moins un groupe aldéhyde et peut être obtenu par la polyalcoxylation d'un aldéhyde **ALD** de formule (I) selon l'une quelconque des revendications 1 à 4 ou d'un aldéhyde de formule (V) selon la revendication 5.

12. Composition durcissable contenant au moins un poly(isocyanate) et au moins un aldéhyde **ALD** de formule (I) selon l'une quelconque des revendications 1 à 4, un aldéhyde de formule (V) selon la revendication 5 ou un aldéhyde de formule (VII) selon la revendication 6.

13. Composition durcie obtenue en faisant réagir une composition durcissable selon la revendication 12 et de l'eau.

14. Procédé de liaison d'un substrat **S1** à un substrat **S2**, qui comprend les étapes de :
i) application d'une composition durcissable selon la revendication 12 à un substrat **S1** ;
ii) mise en contact de la composition appliquée avec un substrat **S2** dans le temps d'assemblage ouvert de la composition ;
ou
i') application d'une composition selon la revendication 12 à un substrat **S1** et à un substrat **S2** ;
ii') mise en contact des compositions appliquées l'une avec l'autre dans le temps d'assemblage ouvert de la composition ;
le substrat **S2** consistant en le même matériau que le substrat **S1** ou en un matériau différent de celui-ci.

15. Article qui a été lié par un procédé selon la revendication 14.
